# EUROPÄISCHE PATENTANMELDUNG

(11) **EP 4 741 486 A1**
(43) Veröffentlichungstag der Anmeldung: **13.05.2026**
(21) Anmeldenummer: 25224515.4
(22) Anmeldetag: 26.06.2020
(51) Int. Cl.: C12M 1/24, C12M 1/00, C12M 3/00, C12M 1/34

(54) **KULTIVIERUNGSSYSTEM UND BEHÄLTERAUFSATZ FÜR EINEN KULTIVIERUNGSBEHÄLTER**

(30) Priorität: 27.06.2019 DE 102019117446
(62) Teilanmeldung aus: 20182588.2
(71) Anmelder: SCHOTT AG, 55122 Mainz (DE)
(72) Erfinder: OTT, Christian, 84028 Landshut (DE); KOLBERG, Christoph, 84028 Landshut (DE)
(74) Vertreter: Schott Corporate IP

(57) **Zusammenfassung**

Die Erfindung betrifft einen Behälteraufsatz, welcher auf den Hals eines Kultivierungsbehälters aufsetzbar ist, um die Öffnung des Kultivierungsbehälters zu verschließen, wobei der Behälteraufsatz eine Innenseite und eine Außenseite aufweist, wobei die Innenseite dem Inneren des Kultivierungsbehälters zugewandt ist und die Außenseite dem Äußeren des Kultivierungsbehälters zugewandt ist, wenn der Behälteraufsatz auf dem Hals des Kultivierungsbehälters aufgesetzt ist, wobei der Behälteraufsatz zumindest eine Sensoreinheit und zumindest eine Abgabeeinheit umfasst, wobei die Sensoreinheit zumindest bereichsweise an der Innenseite des Behälteraufsatzes angeordnet ist, um eine Parametermessung im Inneren des Kultivierungsbehälters zu ermöglichen und wobei die Abgabeeinheit zumindest bereichsweise an der Innenseite des Behälteraufsatzes angeordnet ist, um eine Flüssigkeitsabgabe in das Innere des Kultivierungsbehälters zu ermöglichen.

Weitere Aspekte der Erfindung betreffen ein Kultivierungssystem mit einem solchen Behälteraufsatz sowie ein Verfahren zur Kultivierung biologischen Materials unter Verwendung eines solchen Kultivierungssystems.

## Beschreibung

Die Erfindung betrifft ein Kultivierungssystem mit einem Behälteraufsatz für einen, insbesondere handhaltbaren, Kultivierungsbehälter, z.B. für einen Schüttelkolben.

Bioreaktoren und Schüttelkolben dienen zur Kultivierung von Mikroorganismen, tierischen und pflanzlichen Zellen und eröffnen damit ein breites Anwendungsfeld von Biotech-Produktionsprozessen. Generell besteht ein Bedarf, diese Prozesse weiter zu optimieren. Insbesondere für die Herstellung von Biopharmazeutika werden Verbesserungen der Produktausbeute und damit Gewinnsteigerungen forciert. Zur Steuerung und Kontrolle der Produktionsprozesse, Erhöhung der Produktausbeute sowie der Reduktion von Kosten stehen verschiedene Ansätze zur Verfügung.

Generell kann eine Steuerung und Kontrolle der Prozesse dadurch erfolgen, dass Substrat- und Produktkonzentrationen ermittelt werden. Dies ist allerdings zeitaufwändig und erfordert in der Regel eine ressourcenintensive Offline-Analytik. Bedingt durch den Probenzug ist hiermit zudem ein nicht unerhebliches Kontaminationsrisiko verbunden.

Kommen klassische Bioreaktoren zum Einsatz, kann die Steuerung der Prozesse und damit die Ausbeute dadurch optimiert werden, dass eine Echtzeit-Prozesskontrolle von Schlüsselparametern durchgeführt wird. Zur Erhöhung der Produktausbeute ist insbesondere das in-situ-Monitoring von Parametern, wie der Temperatur, metabolismus- oder produktbildungsrelevanten Stoffen vorteilhaft, ebenso wie eine Regelung von Kultivierungsbedingungen in Echtzeit.

Kommen demgegenüber Schüttelkolben oder andere handhaltbare Kultivierungsbehälter zum Einsatz, bleibt bislang ein Probenzug erforderlich, welcher aufgrund des Handlings bei geöffneter Kultivierungseinheit ein Kontaminationsrisiko birgt. Der Probenzug ist außerdem zeitaufwändig, ebenso wie die Ermittlung von Substrat- und Produktkonzentrationen aufgrund der Offline-Analytik.

Ein Probenzug kann auch bedeuten, dass durch die Unterbrechung der Durchmischung mittels Schüttelinkubator der Stoffaustausch, insbesondere der von gelösten Gasen, kultivierungsinhibierend zum Erliegen kommt. Zusätzlich kann die vielstündige Reaktionszeit bei pH-Korrekturen oder Substratzugaben (feed) stören, welche durch die Dauer der off-line Analytik bedingt ist. Weiterhin steigt mit jedem Öffnen des Schüttelkolbens das Kontaminationsrisiko an.

Durch den Metabolismus entstehen in der Regel pH-senkende Stoffwechselprodukte. Dem wird mitunter versucht, durch Puffern des Mediums entgegenzuwirken. Insbesondere bei der Prozessentwicklung können pH-Kontrollen und Zugaben zur pH-Einstellung bei Erschöpfung der Pufferkapazität erforderlich sein. Mediumsbestandteile sollten zu Beginn der Kultivierung nicht beliebig konzentriert vorgelegt werden, da dies zu einer unerwünschten Substratinhibierung führen kann.

Um die Anzahl von Probenzügen bei Einsatz eines Schüttelkolbens zu reduzieren, gibt es die Ansätze, dass durch den Schüttelkolben hindurch die optische Dichte und die Lumineszenz, z. B. für pH und pO₂, von eingeklebten Spots gemessen wird.

Die optische Güte der Schüttelkolbenwandung (z. B. Transmission, Streuung) schränkt dieses Prinzip jedoch ein. Ferner ist zu beachten, dass die Erfassung des Zellwachstums über die optische Dichte einen konstanten Extinktionskoeffizienten bedingt. Dieser variiert jedoch mit der sich während der Kultivierung ändernden Mediums-Zusammensetzung. Dadurch entsteht ein Unterschied zwischen vitalen und lysierten Zellen. Bei eingeklebten Spots kommen einschränkend noch sog. "Leaching" und "Drift" hinzu.

Die DE202016000554U1 betrifft eine Einweg-Anschlusseinrichtung zum Einsetzen in eine Anschlussöffnung einer Kopfplatte eines Bioreaktors und/oder in eine Anschlussöffnung eines Beutel-Bioreaktors, mit mehreren Durchgängen und einem Befestigungsabschnitt, wobei die Einweg-Anschlusseinrichtung einstückig und aus Kunststoff ausgebildet ist und der Befestigungsabschnitt an einer Außenumfangsfläche eine Befestigungsstruktur zum Befestigen der Einweg-Anschlusseinrichtung in der Anschlussöffnung aufweist.

Die WO2014044612A1 betrifft einen als Einwegelement ausgeführten Reaktortank mit Deckel und/oder im Innenraum befestigte opto-elektronisch lesbare Sensorpatche, einen Reaktor umfassend den Reaktortank und eine Reaktortankaufnahmeperipherie, ihrerseits umfassend eine Reaktortankhalterung und ggf. ein opto-elektronisches Messsystem zum Ablesen von Sensorpatchen, wobei die Reaktortankhalterung an eine Antriebseinheit zur Erzeugung einer rotatorisch-oszillierenden Bewegung des Reaktortanks um eigene mittlere vertikale Achse gekoppelt ist, sowie die Verwendung dieser Vorrichtung zur Kultivierung von Zellen und/oder Mikroorganismen.

Die EP3109314A1 betrifft eine Zellkultivierungsvorrichtung umfassend einen Kulturtank zur Aufnahme einer Kulturlösung, die eine Zelle enthält, ein Wellenelement, das zumindest teilweise in dem Kulturtank angeordnet ist, einen Rührmechanismus, der von dem Wellenelement getragen wird, in dem Kulturtank angeordnet ist und mindestens ein Paar Rührblätter aufweist, die so konfiguriert sind, dass sie um das Wellenelement drehbar sind, und einen Filter, der in Kontakt mit dem Wellenelement angeordnet ist, um die Kulturlösung aus dem Kulturtankanzusaugen und/oder die Kulturlösung dem Kulturtank zuzuführen.

Die CN201658978U betrifft eine Bioexperimentiervorrichtung, die mindestens einen Flaschenhauptkörper umfasst; der Flaschenhauptkörper ist mit einer Flaschenöffnung versehen; über der Flaschenöffnung ist ein Flaschenkorken angeordnet; auf die Flaschenöffnung ist eine Flaschenkappe aufgeschraubt; die innere Seitenwand der Flaschenkappe und die äußere Umfangswand der Flaschenöffnung sind gewindemäßig passend verbunden; die Flaschenkappe drückt den Flaschenkorken auf die Flaschenöffnung; die obere Oberfläche der Flaschenkappe ist mit einer Öffnung versehen, die dazu führt, dass der Flaschenkorken teilweise freigelegt wird; der freigelegte Teil des Flaschenkorkens wird teilweise in ein Lufteinlassrohr, ein Luftauslassrohr und ein Probenentnahmerohr eingeführt; und das obere Ende des Probenentnahmerohrs ist mit einer Dichtungsabdeckung zum Abdichten des Probenentnahmerohrs versehen.

Die WO2018097510A1 betrifft einen Bioreaktionsbehälter umfassend: eine Kulturkammer, die eine Kulturlösung und einen Organismus in ihrem Innenraum aufnimmt und einen offenen oberen Endteil; einen Kammerverschlussteil, der mit dem oberen Endteil der Kulturkammer gekoppelt ist und an einer Seite davon ein Vorsprungsrohr umfasst, das mit dem Innenraum in Verbindung steht; eine Filterkappe, die abnehmbar mit dem Vorsprungsrohr gekoppelt ist und das Vorsprungsrohr öffnet/verschließt einen Gasinjektionsteil, der den Kammerverschlussteil durchdringt und mit dem Innenraum in Verbindung steht, um dem Innenraum ein vorbestimmtes Gas zuzuführen; und einen Säure/Basizität-Einstellungsteil, der an dem Kammerverschlussteil in einem Zustand vorgesehen ist, in dem eine Einstellungslösung zum Einstellen des pH-Wertes der Kulturlösung darin untergebracht ist, und der es ermöglicht, dass die Einstellungslösung durch Luftdruck in den Innenraum abgegeben wird.

Die CN202297585U betrifft einen Tank für die Kultivierung von flüssigen Mikroorganismen umfassend einen Tankkörper und ferner eine Tankkörperhaube, einen Luftkompressor, einen Rührer, eine Flasche mit saurem Material, eine Flasche mit alkalischem Material, einen Temperatursensor und einen pH-Sensor, wobei die Tankkörperhaube eine Endöffnung des Tankkörpers abdeckt; ein Wassereinlass in der Tankkörperhaube ausgebildet ist; eine Polsteröffnung in der Mitte der Tankkörperhaube ausgebildet ist; das hintere Ende des Rührers durch die Polsteröffnung eindringt und die Tankkörperhaube den Rührer axial positioniert; der Temperatursensor und der pH-Sensor sind an der inneren Seitenwand des Tankkörpers angeordnet; eine Auslassöffnung ist im unteren Teil der Seitenwand des Tankkörpers ausgebildet; der Luftkompressor ist durch eine Rohrleitung mit dem inneren Bodenteil des Tankkörpers verbunden; und die Flasche mit dem sauren Material und die Flasche mit dem alkalischen Material sind jeweils durch Rohrleitungen mit dem Inneren des Tankkörpers verbunden.

Die WO1990008816A1 betrifft einen Apparat, eine Vorrichtung und ein Verfahren für die sequentielle Anreicherung eines bestimmten Mikroorganismus bis zu einem Schwellenwert, um seinen Nachweis zu ermöglichen. Die Verwendung der Vorrichtung zur sequentiellen Anreicherung von Mikroorganismen verkürzt die Zeitspanne, die normalerweise für die Anreicherung von Proben benötigt wird, um das Wachstum einer ausreichenden Anzahl von Mikroorganismen zu ermöglichen, um den bestimmten Mikroorganismus von Interesse nachzuweisen.

Die JP2007202542A betrifft ein Zellkulturgefäß bei welchem ein Kulturlösungsreservoirteil am Boden des Kulturlösungsgefäßes angebracht ist und die Spitze eines Abflussrohrs zum Ablassen der Kulturlösung aus dem Inneren des Kulturlösungsgefäßes nach außen des Gefäßes so angeorndet ist, dass es mit der Flüssigkeitsoberfläche des Kulturlösungsreservoirs in Kontakt kommt. Dadurch werden die Blasen aus dem Kulturlösungsreservoir entfernt.

Die WO2018037402A1 betrifft einen Bioreaktor umfassend einen Innenraum und eine perforierte Barriere innerhalb des Gefäßes, durch die eine Flüssigkeit fließen kann, wobei Zellen oder Mikroorganismen die perforierte Barriere nicht passieren können. Die perforierte Barriere unterteilt den Innenraum des Bioreaktors in eine erste Kammer und eine zweite Kammer. In der zweiten Kammer werden Zellen gezüchtet und können durch Rezirkulation der Flüssigkeit, z. B. eines Wachstumsmediums, durch den Bioreaktor perfundiert werden. Verschiedene Einlass- und Auslassöffnungen ermöglichen die Steuerung der Durchflussparameter des Wachstumsmediums.

Die JPH06141850A betrifft einen Kulturtankkörper, der mit einem Rührwerk ausgestattet ist und über entsprechende Pumpen steril mit einem temporären Zellreservoir und einem Abfallflüssigkeitstank verbunden ist. Während der Kultivierung wird das Rühren vorübergehend gestoppt, um die entstandenen Zellaggregate abzusetzen, und eine überstehende Kulturflüssigkeit, die die zu gewinnenden Zellen enthält, wird in den Behälter gesaugt, wo die Kulturflüssigkeit vorübergehend aufbewahrt wird und die an der Behälterwand haftenden Zellaggregate z. B. durch kräftiges Rühren oder die Anwendung von Vibrations-Ultraschallwellen abgelöst und zusammen mit den im Kulturtank verbliebenen Zellaggregaten in den Abfallflüssigkeitstank abgeleitet werden.

Es ist demnach eine Aufgabe der Erfindung, Kultivierungen mit Schüttelkolben oder anderen handhaltbaren Kultivierungsbehältern ebenso leistungsfähig wie Bioreaktoren zu gestalten und dabei zugleich die Anforderungen an die Peripherie zu minimieren, z.B. die Notwendigkeit einer Sterilwerkbank zu vermeiden. Ein genereller Aspekt der Aufgabe der Erfindung ist es, Biotech-Produktionsprozesse kosteneffizienter zu gestalten und eine agilere Anwendung zu ermöglichen.

Hierzu offenbart die Erfindung einen Behälteraufsatz zum Aufsetzen auf einen, insbesondere handhaltbaren, Kultivierungsbehälter, auf welchen im Folgenden noch näher eingegangen wird. Die Erfindung betrifft ferner ein Kultivierungssystem mit einem, insbesondere handhaltbaren, Kultivierungsbehälter zur Aufnahme eines Kulturmediums und einem Behälteraufsatz, welcher auf den Kultivierungsbehälter aufsetzbar ist.

Der Kultivierungsbehälter, welcher vorzugsweise als Schüttelkolben ausgebildet ist, weist einen Hals und eine sich durch den Hals erstreckende Öffnung auf. Vorzugsweise bildet die Öffnung den einzigen Zugang ins Innere des Kultivierungsbehälters, d.h. es handelt sich vorzugsweise nicht etwa um einen zusätzlichen Port an dem Kultivierungsbehälter.

Der Behälteraufsatz ist auf den Hals des Kultivierungsbehälters aufsetzbar, um die Öffnung des Kultivierungsbehälters zu verschließen, wobei insbesondere ein steriler Verschluss erfolgt. Wenn der Behälteraufsatz auf den Hals des Kultivierungsbehälters aufgesetzt ist, ist eine Innenseite des Behälteraufsatzes dem Inneren des Kultivierungsbehälters zugewandt, also insbesondere dem sterilen Bereich zugewandt, und eine Außenseite des Behälteraufsatzes dem Äußeren des Kultivierungsbehälters zugewandt, also insbesondere dem unsterilen Bereich zugewandt.

Der Behälteraufsatz zeichnet sich dadurch aus, dass er zumindest eine Sensoreinheit, insbesondere an einem Port zur Installation einer Sensoreinheit, umfasst und eine Abgabeeinheit, insbesondere an einem Port zur Installation einer Abgabeeinheit, umfasst.

Die umfasste Sensoreinheit ist zumindest bereichsweise an der Innenseite des Behälteraufsatzes angeordnet, um eine Parametermessung im Inneren des Kultivierungsbehälters zu ermöglichen. Die Sensoreinheit kann beispielsweise fest an der Innenseite des Behälteraufsatzes angebracht sein.

Andererseits kann die Sensoreinheit aber auch an der Innenseite des Kultivierungsbehälters anordenbar bzw. installierbar sein, so dass insbesondere ein modularer Austausch möglich ist, z.B. indem eine Sensor-Komponente oder einer Multi-Sensor-Komponente in einem entsprechenden Port des Behälteraufsatzes installiert wird. Hierzu wird insbesondere auf die Deutsche Patentanmeldung 10 2019 117 446.5 verwiesen, welche hiermit durch Referenz inkorporiert wird.

Die umfasste Abgabeeinheit ist ebenfalls zumindest bereichsweise an der Innenseite des Behälteraufsatzes angeordnet, um eine Flüssigkeitsabgabe in das Innere des Kultivierungsbehälters zu ermöglichen. Die Abgabeeinheit kann wiederum beispielsweise fest an dem Behälteraufsatz angebracht sein, insbesondere vollständig oder zumindest teilweise an dessen Innenseite, damit eine Abgabe von Flüssigkeit ins Innere des Behälters ermöglichst wird.

Andererseits kann die Abgabeeinheit aber auch zumindest teilweise, d.h. zumindest mit einem Teilbereich der Abgabeeinheit, an der Innenseite des Kultivierungsbehälters anordenbar bzw. installierbar sein, so dass insbesondere ein modularer Austausch möglich ist, z.B. indem die Abgabeeinheit in einem entsprechenden Port des Behälteraufsatzes installiert wird.

Während mit einer Sensoreinheit in Echtzeit Schlüsselparameter zur Prozesskontrolle erfasst und ggf. verarbeitet werden können, erlaubt eine Abgabeeinheit aktive Korrekturen, z.B. pH-Korrektur oder Feedrate. Liegen Parameterabweichungen vom Sollwert außerhalb der Eingriffsgrenzen, können z.B. aktiv Aktoren angesteuert werden, z. B. pH-Korrektur oder Feedrate. Indem der Behälteraufsatz eine Sensoreinheit und eine Abgabeeinheit umfasst, kann somit eine Integration von Sensorik und Aktoren ermöglicht werden, so dass insbesondere autonome Kultivierungssysteme mit geschlossenen Regelkreisen für optimierte Kultivierungsbedingungen realisierbar sind. Mit der Sensoreinheit und Abgabeeinheit wird somit ein CAP-System (Controlled and Adjusted Process) geschaffen, welches insbesondere den bedarfsorientierten Einsatz von Sensoren und Aktoren in einem System ermöglicht.

Mit der Erfindung, also z.B. mit einem CAP-Kultivierungssystem, welches insbesondere einen handhaltbaren Kultivierungsbehälter oder einen Schüttelkolben umfasst, reduzieren sich die Anforderungen an die Peripherie drastisch, insbesondere gegenüber klassischen Bioreaktoren. Mitunter kann bereits ein Schüttelinkubator und ein Autoklav ausreichend sein. Sogar eine Sterilwerkbank kann ggf. vermieden werden, wobei insbesondere Steril-Konnektoren vorgesehen sein können, wie weiter unten noch näher ausgeführt wird. Die genannten Aspekte steigern die Kosteneffizienz und ermöglichen eine sowohl breitere als auch agile Anwendung. Mit der Erfindung wird es ermöglicht, dass ununterbrochen die Kultivierungsbedingungen im optimalen Bereich gehalten werden. Parameterabweichungen kann instantan entgegengewirkt werden. Die Erfindung stellt ein Kultivierungssystem bereit, dass vergleichbar leistungsstark ist wie ein Bioreaktor.

Der Kultivierungsbehälter kann grundsätzlich jede Form aufweisen, beispielsweise wie ein klassischer Bioreaktor (ggf. mit Stömungsbrechern) mit geraden Seitenwänden geformt sein. Bevorzugt sind jedoch handhaltbare Kultivierungsbehälter, insbesondere Schüttelkolben. Gründe hierfür sind z.B. Verfügbarkeit und Design, wobei ein Designmerkmal die Stoff- und Wärmeübertragung sein kann, die sich insbesondere über die Anzahl an Schikanen beeinflussen lässt.

Der Kultivierungsbehälter weist einen Hals auf, welcher vorzugweise ringförmig, insbesondere zylindrisch, ausgebildet ist. Entsprechend kann der Behälteraufsatz einen ringförmigen, insbesondere zylindrischen, Spalt zur Aufnahme des Halses des Kultivierungsbehälters aufweisen.

Ferner kann der Behälteraufsatz einen Außenkranz aufweisen, welcher der Außenseite des Behälteraufsatzes angehört und den Hals des Kultivierungsbehälters radial umgibt, wenn der Behälteraufsatz auf den Hals des Kultivierungsbehälters aufgesetzt ist. Der Behälteraufsatz kann auch einen Innenvorsprung aufweisen, welcher der Innenseite des Behälteraufsatzes angehört und in die sich durch den Hals des Kultivierungsbehälters erstreckende Öffnung hineinragt, wenn der Behälteraufsatz auf den Hals des Kultivierungsbehälters aufgesetzt ist. Es kann vorgesehen sein, dass der Außenkranz und der Innenvorsprung des Behälteraufsatzes monolithisch ausgebildet sind. Mit anderen Worten ist der Außenkranz insbesondere nicht gegenüber dem Innenvorsprung drehbar ausgebildet.

Der Kultivierungsbehälter kann einen flach ausgebildeten Boden zum Abstellen auf einem Untergrund aufweisen. Der Hals des Kultivierungsbehälters kann z.B. senkrecht nach oben verlaufen, insbesondere, wenn der Kultivierungsbehälter auf dem Untergrund abgestellt ist. Entsprechend kann der Behälteraufsatz senkrecht von oben auf den Hals des Kultivierungsbehälters aufsetzbar sein und vorzugsweise ferner ausgebildet sein, durch die Schwerkraft auf dem Hals des Kultivierungsbehälters zu halten.

Der auf dem Kultivierungsbehälter aufsetzbare Behälteraufsatz weist vorzugsweise eine Anschlagsfläche auf, welche auf dem Kultivierungsbehälter zur Anlage kommt, wenn der Behälteraufsatz auf dem Kultivierungsbehälter aufgesetzt ist. Die Anschlagsfläche befindet sich insbesondere im Inneren eines in dem Behälteraufsatz vorgesehen, insbesondere ringförmigen, Spalts zur Aufnahme des Halses des Kultivierungsbehälters. Mit anderen Worten stützt sich der Behälteraufsatz insbesondere mit seiner Anschlagsfläche auf dem stirnseitigen Ende des Halses ab, wenn der Behälteraufsatz auf dem Kultivierungsbehälter aufgesetzt ist.

Der Kultivierungsbehälter weist insbesondere ein Volumen von weniger als 2801 Milliliter auf, vorzugsweise von weniger als 1801 Milliliter, noch bevorzugter von weniger als 501 Milliliter, nochmals bevorzugter von weniger als 251 Milliliter und besonders bevorzugt von weniger als 126 Milliliter.

Ferner kann der Kultivierungsbehälter einen Durchmesser von weniger als 22 Zentimeter oder von weniger als 21 Zentimeter aufweisen, vorzugsweise von weniger als 14 Zentimeter oder von weniger als 12 Zentimeter, besonders bevorzugt von weniger als 9 Zentimeter oder von weniger als 8 Zentimeter.

Der Kultivierungsbehälter kann eine Höhe von weniger als 31 Zentimeter aufweisen, vorzugsweise von weniger als 23 Zentimeter, nochmals bevorzugter von weniger als 19 Zentimeter, besonders bevorzugt von weniger als 17 Zentimeter, ganz besonders bevorzugt von weniger als 14 Zentimeter.

Der auf dem Kultivierungsbehälter aufsetzbare Behälteraufsatz hat vorzugsweise eine von der Anschlagsfläche gemessene Höhe von weniger 10 als Zentimeter, vorzugsweise von weniger als 5 Zentimeter, noch bevorzugter von weniger als 4 Zentimeter, nochmals bevorzugter von weniger als 3 Zentimeter, besonders bevorzugt von weniger als 2 Zentimeter. Mit anderen Worten führt der Behälteraufsatz im aufgesetzten Zustand zu einer maximalen Erhöhung des Kultivierungsbehälters um die angegeben Werte.

Wie bereits beschrieben umfasst der Behälteraufsatz vorzugsweise eine oder mehrere Abgabeeinheiten, welche zur Steuerung von Prozessparametern dienen. Die zumindest eine Abgabeeinheit des Behälteraufsatzes umfasst ein Reservoir zur Flüssigkeitsspeicherung, wobei das Reservoir einen unteren Reservoirabschnitt mit einer Auslauföffnung zur Flüssigkeitsabgabe und vorzugsweise einen oberen Reservoirabschnitt mit eine Nachströmungsöffnung zum Nachströmen von Gas aufweist.

Der untere Reservoirabschnitt ist vorzugsweise an der Innenseite des Behälteraufsatzes angeordnet oder anordenbar, um eine Flüssigkeitsabgabe durch die Auslauföffnung in das Innere des Kultivierungsbehälters zu ermöglichen und der obere Reservoirabschnitt ist vorzugsweise an der Außenseite des Behälteraufsatzes angeordnet oder anordenbar und besonders bevorzugt breiter ausgebildet, um einen Anschlag zu bilden, wenn die Abgabeeinheit und/oder das Reservoir an einem Port des Behälteraufsatzes installiert wird.

Der Behälteraufsatz und/oder die Abgabeeinheit kann eine, insbesondere einen Schlauch umfassende, Verbindung zwischen der Nachströmungsöffnung des oberen Reservoirabschnitts und der Innenseite des Behälteraufsatzes aufweist, um ein, insbesondere steriles, Nachströmen von Gas aus dem Inneren des Kultivierungsbehälters zu ermöglichen, wenn der Behälteraufsatz auf dem Hals des Kultivierungsbehälters aufgesetzt ist.

Das Reservoir, insbesondere der obere Reservoirabschnitt, kann ferner eine Nachfüllöffnung zum Nachfüllen von Flüssigkeit aufweisen, wobei der Behälteraufsatz und/oder die Abgabeeinheit vorzugsweise eine, insbesondere einen Schlauch umfassende, Verbindung zwischen der Nachfüllöffnung des Reservoirs und einem an der Außenseite des Behälteraufsatzes angeordneten oder anordenbaren Ventil aufweist, um ein, insbesondere steriles, Nachfüllen von Flüssigkeit in das Reservoir zu ermöglichen. Der Behälteraufsatz und/oder die Abgabeeinheit kann ferner eine, z.B. radial außen angebrachte, Halterung zur lösbaren Fixierung des Ventils aufweisen.

Um eine, insbesondere sterile, Zugabe oder Entnahme aus dem Inneren des Kultivierungsbehälters zu ermöglichen, wenn der Behälteraufsatz auf dem Hals des Kultivierungsbehälters aufgesetzt ist, kann der Behälteraufsatz außerdem eine, insbesondere einen Schlauch umfassende, Verbindung zwischen der Innenseite des Behälteraufsatzes und einem an der Außenseite des Behälteraufsatzes angeordneten oder anordenbaren Ventil aufweisen. Auch diesbezüglich kann der Behälteraufsatz eine, z.B. radial außen angebrachte, Halterung zur lösbaren Fixierung des Ventils aufweisen.

Die zumindest eine Sensoreinheit des Behälteraufsatzes kann insbesondere als Biosensoreinheit zur analytspezifischen Parametermessung ausgebildet sein. Eine analytspezifische Parametermessung kann implizieren, dass eine bestimmte Messgröße, der Analyt, selektiv erfasst wird, insbesondere also nicht gleichzeitig mehrere Parameter, z. B. verschiedene Analyten, miterfasst erfasst werden.

Die zumindest eine oder eine weitere Sensoreinheit des Behälteraufsatzes, welche insbesondere nicht als Biosensoreinheit ausgebildet ist, kann andererseits auch als Luminophoreneinheit zur lumineszenzbasierten Parametermessung ausgebildet sein. Bei der lumineszenbasierten Parametermessung kann ein Analyt-abhängiges Signalquenching erfolgen. Die Anregungswellenlänge kann durch Wechselwirkung mit der Messgröße wellenlängen- und/oder phasenverschoben werden.

Die zumindest eine oder eine weitere Sensoreinheit kann auch als Wechselfeldeinheit zur dielektrizitätsbasierten Parametermessung ausgebildet sein. Verschieben sich bei Analyten im elektrischen Feld Ladungen, so wird ein Dipol induziert. Bei einer dielektrizitätsbasierten Messung kann diese frequenzabhängige Wechselwirkung in einer Sensoreinheit genutzt werden.

Die zumindest eine oder eine weitere Sensoreinheit kann zudem auch als Transistoreinheit zur feldeffektbasierten Parametermessung ausgebildet sein. Dabei können ionische Analyten bei einem Halbleiterelement eine Leitfähigkeit erzeugende Spiegelladung bewirken, insbesondere, indem sie sich ionenselektiv an einen Sensorchip reversibel anlagern.

In Bezug auf die Sensoreinheit wird auf die Deutsche Patentanmeldung 10 2019 117 446.5 verwiesen. Insbesondere kann demnach die zumindest eine Sensoreinheit an einem vorderen Gehäuseabschnitt einer Sensor-Komponente, insbesondere einer Multi-Sensor-Komponente, angeordnet oder anordenbar sein, derart, dass die zumindest eine Sensoreinheit an der Innenseite des Behälteraufsatzes anordenbar ist, indem die Sensor-Komponente an einem Port des Behälteraufsatzes installiert wird, um eine Parametermessung im Inneren des Kultivierungsbehälters zu ermöglichen, wenn der Behälteraufsatz auf dem Hals des Kultivierungsbehälters aufgesetzt ist. Im Fall einer Multi-Sensor-Komponente können mehrere Sensoreinheiten zugleich installierbar sein.

Vorzugsweise umfasst der Behälteraufsatz eine Mehrzahl von Sensor-/Abgabeeinheiten, insbesondere an entsprechenden Ports, besonders bevorzugt selektiv zumindest eine Sensoreinheit sowie zumindest zwei Abgabeeinheiten.

Neben dem vorstehend beschriebenen Kultivierungssystem mit Kultivierungsbehälter und Behälteraufsatz betrifft die Erfindung auch den Behälteraufsatz zum Aufsetzen auf einen, insbesondere handhaltbaren, Kultivierungsbehälter, vorzugsweise auf einen Schüttelkolben.

Der Behälteraufsatz umfasst eine Innenseite, welche dem Inneren des Kultivierungsbehälters zugewandt ist, wenn der Behälteraufsatz auf dem Kultivierungsbehälter aufgesetzt ist und eine Außenseite, welche dem Äußeren des Kultivierungsbehälters zugewandt ist, wenn der Behälteraufsatz auf dem Kultivierungsbehälter aufgesetzt ist.

Ferner umfasst der Behälteraufsatz zumindest eine Sensoreinheit, insbesondere an einem Port zur Installation einer Sensoreinheit, und zumindest eine Abgabeeinheit, insbesondere an einem Port zur Installation einer Abgabeeinheit. Die Sensoreinheit ist zumindest bereichsweise an der Innenseite des Behälteraufsatzes angeordnet, um eine Parametermessung im Inneren des Kultivierungsbehälters zu ermöglichen. Auch die Abgabeeinheit ist zumindest bereichsweise an der Innenseite des Behälteraufsatzes angeordnet, um eine Flüssigkeitsabgabe in das Innere des Kultivierungsbehälters zu ermöglichen.

Der Behälteraufsatz kann einen ringförmigen, insbesondere zylindrischen, Spalt zur Aufnahme eines Halses des Kultivierungsbehälters aufweisen. Ferner kann der Behälteraufsatz einen Außenkranz aufweisen, welcher den Hals eines Kultivierungsbehälters radial umgibt, wenn der Behälteraufsatz auf einem Kultivierungsbehälter aufgesetzt ist. Außerdem kann der Behälteraufsatz einen Innenvorsprung aufweisen, welcher in den Hals eines Kultivierungsbehälters hineinragt, wenn der Behälteraufsatz auf einem Kultivierungsbehälter aufgesetzt ist. Der Außenkranz und der Innenvorsprung können monolithisch ausgebildet sein.

Der Behälteraufsatz ist insbesondere ausgebildet, senkrecht von oben auf einen Kultivierungsbehälter aufgesetzt zu werden und ist vorzugsweise ausgebildet, durch die Schwerkraft auf dem Kultivierungsbehälter zu halten.

Der Behälteraufsatz weist vorzugsweise eine Anschlagsfläche auf, welche auf dem Kultivierungsbehälter zur Anlage kommt, wenn der Behälteraufsatz auf dem Kultivierungsbehälter aufgesetzt ist, wobei sich die Anschlagsfläche insbesondere im Inneren eines in dem Behälteraufsatz vorgesehen, insbesondere ringförmigen, Spalts zur Aufnahme des Halses des Kultivierungsbehälters befindet.

Der auf dem Kultivierungsbehälter aufsetzbare Behälteraufsatz hat vorzugsweise eine von der Anschlagsfläche gemessene Höhe von weniger 10 als Zentimeter, vorzugsweise von weniger als 5 Zentimeter, noch bevorzugter von weniger als 4 Zentimeter, nochmals bevorzugter von weniger als 3 Zentimeter, besonders bevorzugt von weniger als 2 Zentimeter.

Die zumindest eine Abgabeeinheit des Behälteraufsatzes umfasst ein Reservoir zur Flüssigkeitsspeicherung, wobei das Reservoir einen unteren Reservoirabschnitt mit einer Auslauföffnung zur Flüssigkeitsabgabe und vorzugsweise einen oberen Reservoirabschnitt mit eine Nachströmungsöffnung zum Nachströmen von Gas aufweist.

Der untere Reservoirabschnitt ist vorzugsweise an der Innenseite des Behälteraufsatzes angeordnet oder anordenbar, um eine Flüssigkeitsabgabe durch die Auslauföffnung in das Innere des Kultivierungsbehälters zu ermöglichen. Der obere Reservoirabschnitt ist vorzugsweise an der Außenseite des Behälteraufsatzes angeordnet oder anordenbar und besonders bevorzugt breiter ausgebildet, um einen Anschlag zu bilden, wenn die Abgabeeinheit und/oder das Reservoir an einem Port des Behälteraufsatzes installiert wird.

Der Behälteraufsatz und/oder die Abgabeeinheit kann eine Verbindung zwischen der Nachströmungsöffnung des oberen Reservoirabschnitts und der Innenseite des Behälteraufsatzes aufweisen, um ein Nachströmen von Gas aus dem Inneren des Kultivierungsbehälters zu ermöglichen, wenn der Behälteraufsatz auf dem Kultivierungsbehälter aufgesetzt ist.

Ferner kann das Reservoir eine Nachfüllöffnung zum Nachfüllen von Flüssigkeit aufweisen, wobei der Behälteraufsatz und/oder die Abgabeeinheit vorzugsweise eine Verbindung zwischen der Nachfüllöffnung des Reservoirs und einem an der Außenseite des Behälteraufsatzes angeordneten oder anordenbaren Ventil aufweist, um ein Nachfüllen von Flüssigkeit in das Reservoir zu ermöglichen. Der Behälteraufsatz und/oder die Abgabeeinheit kann eine Halterung zur lösbaren Fixierung des Ventils aufweisen.

Um eine Zugabe oder Entnahme aus dem Inneren des Kultivierungsbehälters zu ermöglichen, wenn der Behälteraufsatz auf dem Kultivierungsbehälter aufgesetzt ist, kann der Behälteraufsatz ferner eine Verbindung zwischen der Innenseite des Behälteraufsatzes und einem an der Außenseite des Behälteraufsatzes angeordneten oder anordenbaren Ventil aufweisen. Auch hierzu kann der Behälteraufsatz eine Halterung zur lösbaren Fixierung des Ventils umfassen.

Die zumindest eine Sensoreinheit des Behälteraufsatzes kann als Biosensoreinheit zur analytspezifischen Parametermessung ausgebildet sein. Andererseits kann die zumindest eine oder eine weitere Sensoreinheit des Behälteraufsatzes, welche insbesondere nicht als Biosensoreinheit ausgebildet ist, als Luminophoreneinheit zur lumineszenzbasierten Parametermessung ausgebildet sein. Die zumindest eine oder eine weitere Sensoreinheit kann auch als Wechselfeldeinheit zur dielektrizitätsbasierten Parametermessung ausgebildet sein. Die zumindest eine oder eine weitere Sensoreinheit kann zudem auch als Transistoreinheit zur feldeffektbasierten Parametermessung ausgebildet sein. Insbesondere bezugnehmend auf die Deutsche Patentanmeldung 10 2019 117 446.5 kann die zumindest eine Sensoreinheit an einem vorderen Gehäuseabschnitt einer Sensor-Komponente angeordnet oder anordenbar sein, derart, dass die zumindest eine Sensoreinheit an der Innenseite des Behälteraufsatzes anordenbar ist, indem die Sensor-Komponente an einem Port des Behälteraufsatzes installiert wird.

In einer bevorzugten Ausführungsform weist der Behälteraufsatz eine Mehrzahl von Sensor-/Abgabeeinheiten, insbesondere an entsprechenden Ports, auf, besonders bevorzugt zumindest eine Sensoreinheit sowie zumindest zwei Abgabeeinheiten.

Die Erfindung betrifft ferner auch eine Abgabeeinheit zur Installation an einem Port eines Behälteraufsatzes, insbesondere wie vorstehend beschrieben, wobei die Abgabeeinheit ein Reservoir zur Flüssigkeitsspeicherung umfasst, wobei das Reservoir einen unteren Reservoirabschnitt mit einer Auslauföffnung zur Flüssigkeitsabgabe und vorzugsweise einen oberen Reservoirabschnitt mit einer Nachströmungsöffnung zum Nachströmen von Gas aufweist.

Das Reservoir kann eine Nachfüllöffnung zum Nachfüllen von Flüssigkeit aufweisen, wobei auch eine Verbindung zwischen der Nachfüllöffnung des Reservoirs und einem Ventil vorhanden sein kann, um ein, insbesondere steriles, Nachfüllen von Flüssigkeit in das Reservoir zu ermöglichen.

Die Flüssigkeiten in den Reservoirs und dem Medium sind bevorzugt so aufeinander abgestimmt, dass damit die Bandreite der Kultivierungen im lebenswissenschaftlichen Bereich abdeckbar ist. Beispielsweise kann eine Feedlösung im Reservoir die für die Produktbildung erforderlichen Substrate, wie z. B. Saccharide, beinhalten. Da durch den Zellmetabolismus pH-Wert senkende Mediumsbestandteile, wie z. B. organische Säuren entstehen können, kann vorzugsweise eine pH-Wert hebende, z.B. NaOH-haltige, Korrekturlösung vorgesehen sein. Es kann somit eine pH-Korrekturlösung im Reservoir vorgesehen sein. Mit einer pH-Korrekturlösung im Reservoir kann insbesondere ein Puffersystem mit dem Medium gebildet sein, z.B. bildet Zellmetabolismus CO₂ und damit H₂CO₃ und durch HCO₃-Korrekturlösung ist ein Puffersystem mit nachgeregelter Pufferkapazität und stabilem pH-Wert gebildet.

Ferner kann auch eine pH-Korrektur enthaltende Feedlösung vorgesehen sein. Insbesondere über Stöchiometrie kann sich ergeben, wieviel pH-senkende Substanzen durch die Verstoffwechselung des Feeds gebildet wird. Entsprechend kann sich die pH-Korrektur mit der Feedlösung, unter Berücksichtigung der Substratumsatzrate und Pufferwirkung im Medium, mitdosieren lassen. Dies ermöglicht etwa, dass über Dosierung mit einem ersten Reservoir sich z.B. Biomasse bilden lässt und über Dosierung mit einem weiteren Reservoir die Produktbildung sich optimieren lässt.

Die Erfindung betrifft demnach ferner ein Verfahren zur Vermehrung oder Kultivierung biologischen Materials, vorzugsweise zur Herstellung von Pharmazeutika, insbesondere Biopharmazeutika, mit den folgenden Verfahrensschritten: Bereitstellen eines Kultivierungssystems, insbesondere wie vorstehend beschrieben, umfassend einen Kultivierungsbehälter und zumindest ein Reservoir mit jeweils einer Auslauföffnung zur Flüssigkeitsabgabe in das Innere des Kultivierungsbehälters, Bereitstellen einer Feedlösung, insbesondere enthaltend die für die Produktbildung erforderichen Substrate, z.B. Saccharide, wobei die Feedlösung vorzugsweise in dem Reservoir bereitgestellt wird, Bereitstellen einer pH-Korrekturlösung, insbesondere zum Anheben des pH-Werts, z.B. enthaltend NaOH, wobei die pH-Korrekturlösung vorzugsweise in einem weiteren Reservoir bereitgestellt wird und/oder in der Feedlösung enthaltend bereitgestellt wird.

Die Erfindung betrifft ferner noch ein Verfahren zur Vermehrung oder Kultivierung biologischen Materials, vorzugsweise zur Herstellung von Pharmazeutika, insbesondere Biopharmazeutika, mit den folgenden Verfahrensschritten: Bereitstellen mehrerer Kultivierungssysteme, insbesondere wie vorstehend beschrieben, Verbinden der mehreren Kultivierungssysteme jeweils über einen Zugabe-/Entnahmeschlauch mit einem Pumpenmodul und vorzugsweise Automatisiertes Inokulieren der mehreren Kultivierungssysteme.

Eine erweiterte Verwendung des beschriebenen Kultivierungssystems kann insbesondere zur Modularisierung und Prozessintensivierung dienlich sein. Es können insbesondere mehrere der beschriebenen Kultivierungssysteme eingesetzt werden, wobei jedes einzelne ein standardisiertes Bioprozessmodul repräsentiert, welche eine flexible Anpassung an gewünschte Anforderungen ermöglicht. Beispielhaft dienen diese, neben parallelen Kultivierungen, zur Umsetzung von Strategien zur Prozessintensivierung, welche eine effiziente Steigerung von Raum-Zeit-Ausbeuten beinhalten. Vorzugsweise wird dies erreicht, indem ein klassisches Kryostock-Vail durch eine Kryostockspritze ersetzt wird, mit welcher ein vorbereitetes Kultivierungssystem, direkt über einen Zugabe-/Entnahmeschlauch (genauer: ein damit verbundenes Ventil), insbesondere steril sicher, angeimpft wird. Branchenübliche Handlings- und Peripherieanforderungen können dadurch in vorteilhafter Weise entfallen. Im Rahmen eines Controlled and Adjusted Processes (CAP) in einem Kultivierungssystem der beschriebenen Art kann insbesondere die Vitalisierung und log. Proliferation der Zellen ermöglicht werden. Über einen Zugabe-/Entnahmeschlauch (Transferschlauch) und ggf. ein Pumpenmodul können weitere Kultivierungssysteme automatisiert inokuliert werden. Hierdurch können z.B. verschiedene personalisierte Biopharmazeutika in einem Inkubationsschüttler prozessiert werden. Werden weitere Kultivierungssysteme über einen automatisierten Transfer miteinander verbunden und in das initiale Bioprozessmodul Medium entsprechend nachdosiert, lassen sich somit kontinuierliche Bioprozesse und damit die Prozessintensivierung realisieren. Selbst im Kontaminationsfall ist der Verlust, gegenüber konventionellen Bioprozessen minimal.

Nachfolgend werden einige spezielle, nicht abschließend zu verstehende Ausführungsbeispiele der Erfindung Bezug nehmend auf die beigefügten Zeichnungen erläutert. Es zeigen:
- Fig. 1: eine dreidimensionale Ansicht eines Kultivierungssystems,
- Fig. 2: weitere dreidimensionale Ansichten des Kultivierungssystems aus Fig. 1,
- Fig. 3: eine Schnittansicht des Kultivierungssystems aus Fig. 1,
- Fig. 4: eine weitere Schnittansicht des Kultivierungssystems aus Fig. 1,
- Fig. 5: eine dreidimensionale Ansicht eines Kultivierungssystems,
- Fig. 6: eine dreidimensionale Ansicht eines Behälteraufsatzes,
- Fig. 7: eine dreidimensionale Ansicht eines Elektronikmoduls,
- Fig. 8: dreidimensionale zweier Abgabeeinheiten.

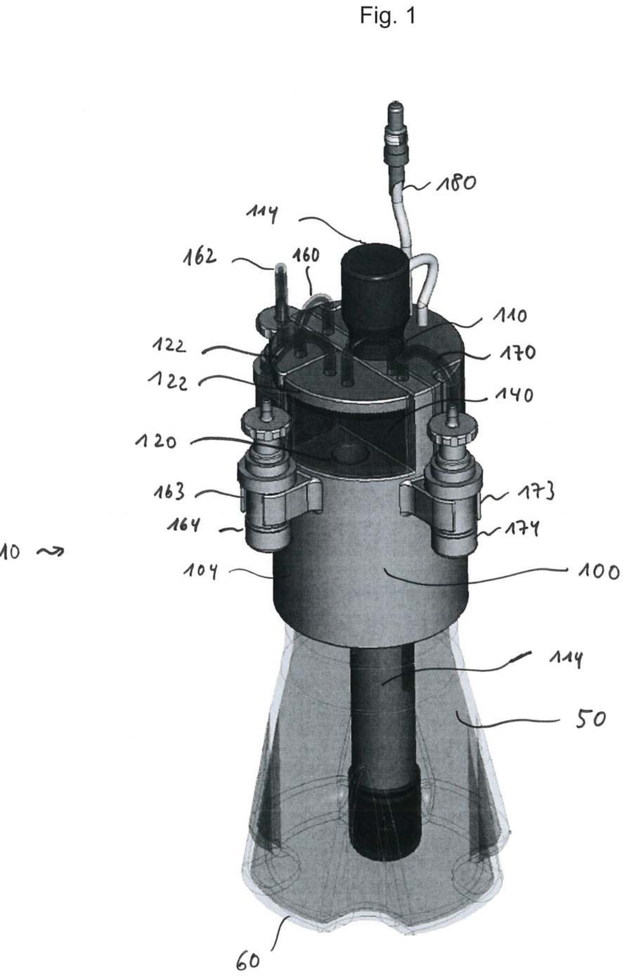

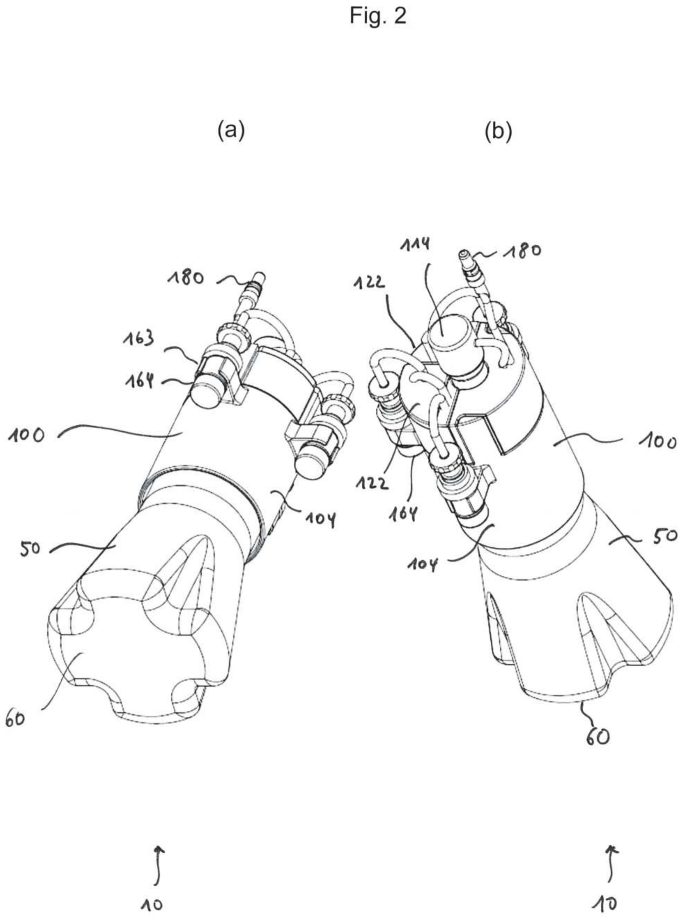

Bezugnehmend auf Fig. 1 bis 3 umfasst ein Kultivierungssystem 10 einen Kultivierungsbehälter 50, insbesondere aus Glas oder Polymer, und einen Behälteraufsatz 100.

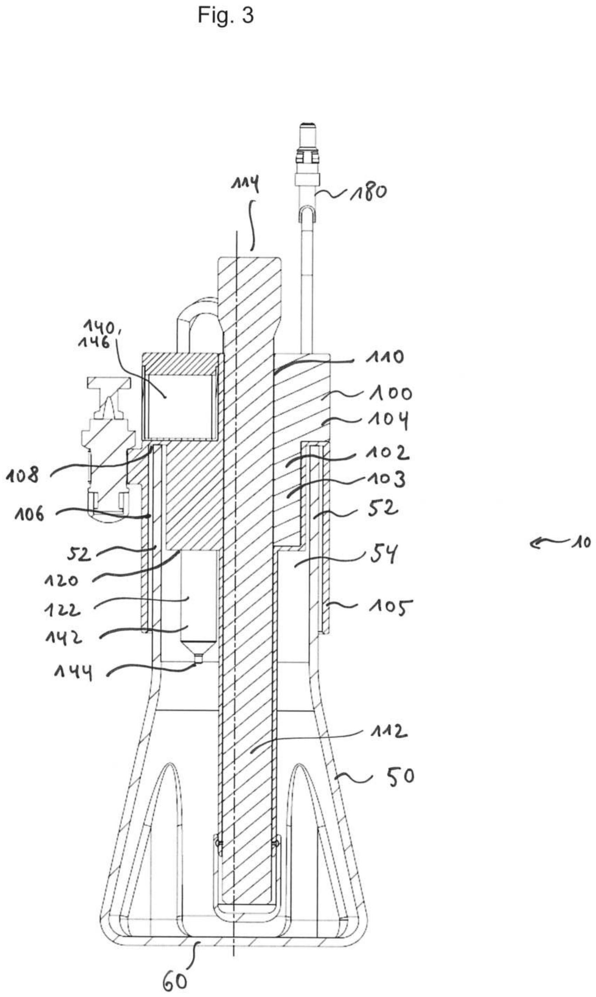

Der Behälteraufsatz 100 ist senkrecht von oben auf dem Hals 52 (siehe Fig. 3) des Kultivierungsbehälters 50 aufgesetzt, wobei der Kultivierungsbehälter 50 einen flachen Boden 60 aufweist, um auf einer Unterlage abgestellt werden zu können, so dass der Hals 52 des Behälters senkrecht nach oben ragt.

Der Behälteraufsatz 100 weist eine Innenseite 102 sowie eine Außenseite 104 auf, wobei die Innenseite 102 in die sich durch den Hals 52 erstreckende Öffnung 54 des Kultivierungsbehälters 50 hineinragt und damit dem Inneren des Behälters 50 zugewandt ist und die Außenseite 104 dem Äußeren des Behälters 50 zugewandt ist.

Der Behälteraufsatz 100 weist einen ringförmigen Spalt 106 auf in welchen der Hals 52 des Kultivierungsbehälters 50 hineinragt. Auf diese Weise wird ein steriler Verschluss des Kultivierungsbehälters 50 ermöglicht. Der Spalt 106 trennt dabei den der Außenseite 104 angehörenden Außenkranz 105 von dem der Innenseite 102 angehörenden Innenvorsprung 103 des Behälteraufsatzes 100, dessen Grundkörper monolithisch, d.h. einstückig ausgebildet ist, so dass insbesondere der Außenkranz 105 und der Innenvorsprung 103 aus einem Materialstück geformt sind.

In dem dargestellten Beispiel umfasst der Behälteraufsatz einen Port 110 zur Installation einer Sensoreinheit 112, welche in diesem Fall in einer modular an dem Port 110 installierbaren Multi-Sensor-Komponente 114 untergebracht ist, sowie zwei Ports 120 zur Installation zweier Abgabeeinheiten 122.

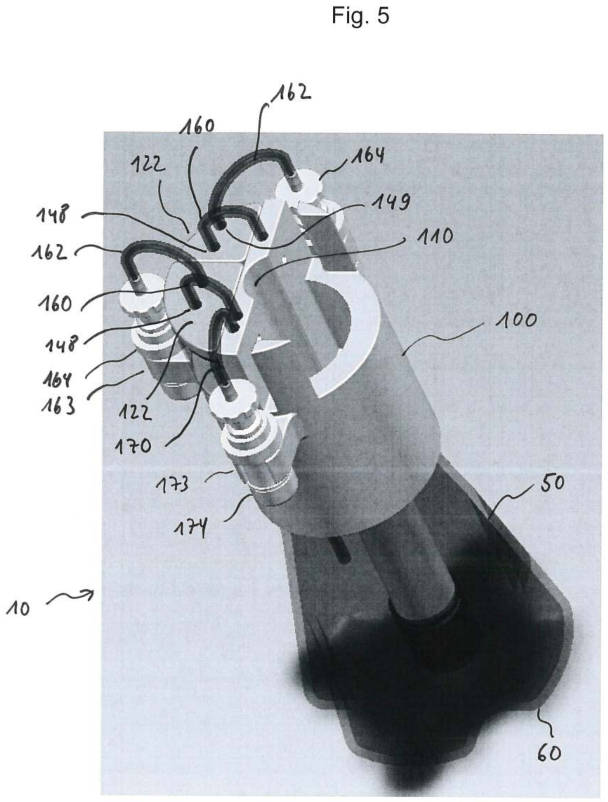

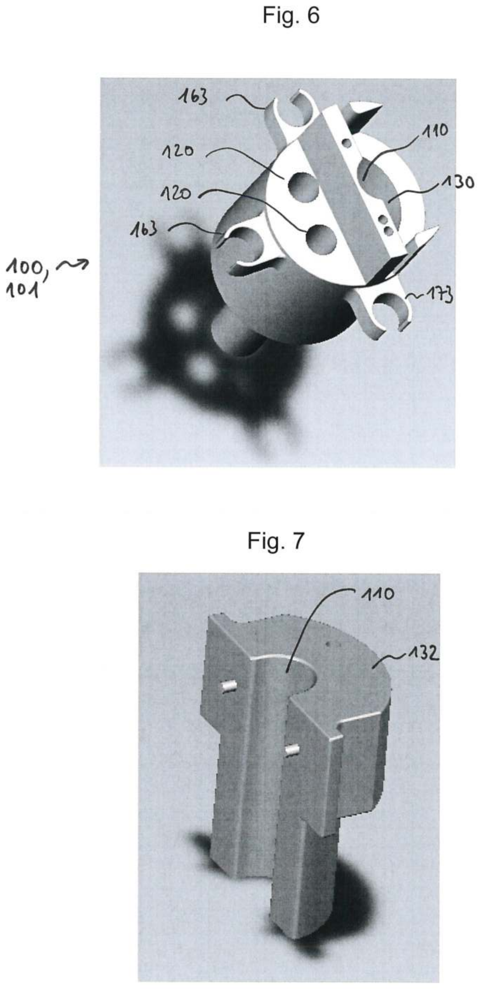

Anhand der Fig. 5 bis 8 ist zu erkennen, dass das Kultivierungssystem 10 vorzugsweise modular ausgebildet ist, so dass z.B. die Sensoreinheit 112 und/oder die Abgabeeinheit 122 modular an dem Behälteraufsatz 100 installierbar sind. Fig. 6 zeigt etwa einen Behälteraufsatz 100 der als Grundkörper 101 ausgebildet ist und Ports 110, 120 zur Installation von Sensoreinheiten und Abgabeeinheiten umfasst. Der gezeigte Behälteraufsatz weist zudem einen Port 130 zur Installation eines Elektronikmoduls 132 auf (siehe Fig. 7), welches seinerseits, wiederum einen Teil des Ports 110 zur Installation einer Sensoreinheit 112 bzw. einer (Multi-)Sensor-Komponente 114 bildet.

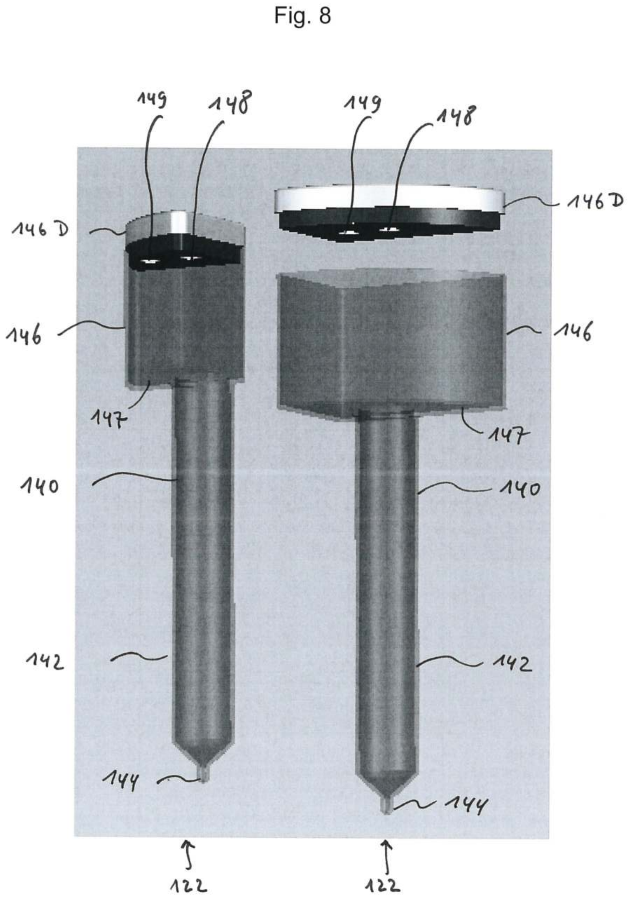

Bezugnehmend auf Fig. 8 umfasst die Abgabeeinheit 122 des Behälteraufsatzes 100 ein Reservoir 140, insbesondere für Feed- und pH-Einstellungsreagenz. Das Reservoir 140 umfasst einen unteren Reservoirabschnitt 142, an dem sich eine Auslauföffnung 144 befindet, und einem oberen Reservoirabschnitt 146, an dem sich eine Nachströmungsöffnung 148 zum Nachströmen von Gas befindet. Der obere Reservoirabschnitt 146 ist breiter als der untere Reservoirabschnitt 142 und bildet dadurch einen Anschlag 147 für die modulare Installation der Abgabeeinheit 122. Der obere Reservoirabschnitt 146 weist zudem eine Nachfüllöffnung 149 auf, um Flüssigkeit in das Reservoir 140 nachzufüllen.

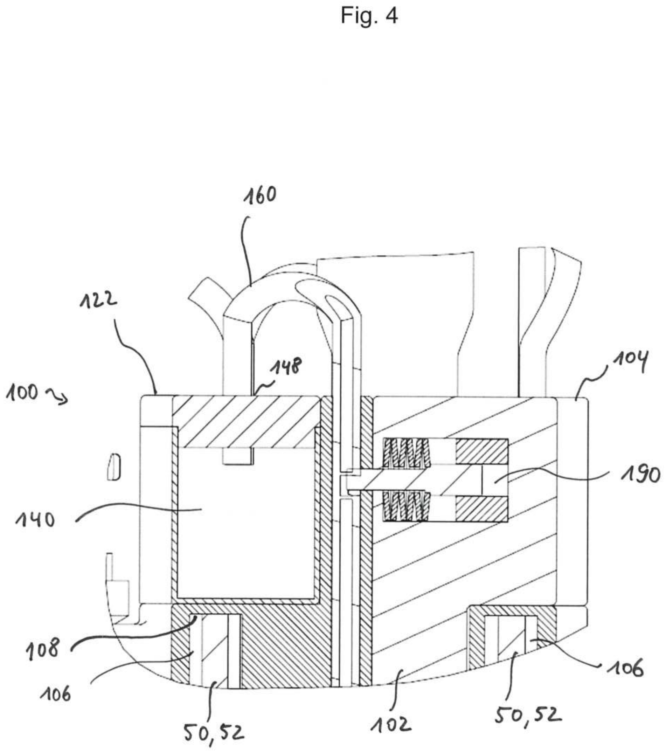

Wie am besten anhand von Fig. 4 und Fig. 5 zu erkennen ist, ist ein Schlauch 160 vorgesehen, welcher von der Nachströmungsöffnung 148 zur Innenseite 102 des Behälteraufsatzes 100 und damit ins Innere des Kultivierungsbehälters 50 führt. Dies ermöglicht ein steriles Nachströmen von Gas aus dem Behälterinneren. Außerdem wird hierdurch eine druckregulierte Dosierung der Feed- bzw. pH-Einstellung ermöglicht, wie weiter unten noch ausgeführt wird.

Ein weiterer Schlauch 162 ist vorgesehen, um die Nachfüllöffnung 149 mit einem an einer Halterung 163 angebrachten Ventil 164 zu verbindet, so dass das Reservoir steril nachgefüllt werden kann (Luer-Lock-Ventile zur sterilen Reservoirbefüllung). Außerdem ist ein, insbesondere als Zugabe-/Entnahmeschlauch ausgebildeter, Schlauch 170 vorgesehen, welcher von einem außen an einer Halterung 173 angebrachten Ventil 174 an die Innenseite des Behälteraufsatzes 100 führt, um eine sterile, Zugabe oder Entnahme aus dem Inneren des Kultivierungsbehälters 50 zu ermöglichen (Luer-Lock-Ventil zur sterilen Inokulierung und/oder Entnahme).

In einer Weiterbildung der Erfindung (nicht abgebildet) kann ggf. eine weitere an die Innenseite des Behälteraufsatzes führende Zugabe- und/oder Entnahmeöffnung insbesondere mit einem Schlauch (Zugabe-/Entnahmeschlauch), vorzugsweise einem Ventil, welches ggf. an einer Halterung befestigbar sein kann, und besonders bevorzugt in Verbindung mit einer Transferpumpe vorgesehen sein, insbesondere um eine Mehrzahl von Kultivierungsystemen 10 zu koppeln. Eine Transferpumpe kann als Modul ausgebildet sein, welches mehrere (z.B. vier) Peristaltikpumpen umfasst, welche beispielsweise auf einem Grundkörper angeordnet sein können, derart, dass die Anordnung entsprechend einem Kultivierungssystem bzw. einem Kultivierungsbehälter auf eine Schüttelkolbenhalterung passt.

Das erfindungsgemäße Kultivierungssystem 10 wird auch als CAP-System bezeichnet (Controlled and Adjusted Processes). CAP bedeutet, dass unabhängig von Form des Kultivierungsgefäßes 50 sowohl die In-Situ-Sensorik aus auch die Aktoren in dem als Kappe ausgebildeten Behälteraufsatz 100 integriert sind. Somit entsteht ein geschlossener Regelkreis, mit dem in Echtzeit die Schlüsselprozessparameter steril und sicher im optimalen Bereich gehalten werden. Für die In-Situ-Sensorik eignet sich insbesondere eine Multi-Sensor-Komponente gemäß der Deutschen Patentanmeldung 10 2019 117 446.5.

Mit dem erfindungsgemäßen CAP-Kultivierungssystem reduzieren sich in vorteilhafter Weise die Anforderungen an die Peripherie gegenüber klassischen Bioreaktoren. Beispielsweise um eine Sterilwerkbank vermeiden zu können kann vorgesehen sein, dass das Ventil 174 mit einer Spritze hierfür einen Steril-Konnektor bildet. In dieser Ausführung kann unter der Schutzkappe von Ventil und Spritze jeweils eine Abdeckfolie mit Lasche vorgesehen sein. Die Konnektorseiten können desinfiziert und aneinandergedrückt werden. Ventil und Spritze können beispielsweise eine Male/Female-Ausführung aufweisen, mit der die Abdeckfolien aneinanderlegend über die Laschen kontaminationsfrei herausgezogen werden können, so dass der Transferweg offen ist. Das gleiche Prinzip kann für die Verbindung über die Ventile 164 der Reservoirs vorgesehen sein. Mit anderen Worten kann allgemein zumindest ein Ventil 164, 174 als Steril-Konnektor ausgebildet sein, z.B. mit einer Abdeckfolie verschlossen sein, um eine sterile Verbindung zu ermöglichen. Hierdurch kann z.B. auch nach Lagerung bei z.B. -80°C die Inokulation über eine Ausführung als Spritze, oder über den Transfer mittels sterilem Fluid / Luft ermöglicht werden.

Das Kultivierungsgefäß 50 an sich, kann wie ein klassischer Bioreaktor (ggf. mit Stömungsbrechern) mit geraden Seitenwänden geformt sein. Durch Design und Verfügbarkeit bevorzugt sind Schüttelkolben. Ein Designmerkmal ist die Stoff- und Wärmeübertragung, die sich insbesondere über die Anzahl an Schikanen beeinflussen lässt.

Das CAP-System kann mit einem Autoklav und mit einer Sterilwerkbank für die Kultivierung vorbereitet werden. Darüber hinaus, ist dies mit der vorliegenden Erfindung in vorteilhafter Weise auch ohne Sterilarbeitsplatz möglich.

Bei Verwendung von Sterilwerkbänken und Autoklaven kann das Medium in einem Standardschüttelkolben 50 vorgelegt und die CAP-Basis 101 und Reservoirs 140 aufgesteckt. Die CAP-Basis 101 ist temperaturstabil bis 141°C, ggf. einschließlich der bereits genannten Schläuche 160, 162, 170, welche z.B. aus PP sein können, Reservoirs 140 bzw. Abgabeeinheiten 122 und Sensor-Komponenten 114 oder Sensoreinheiten 112. Der Behälteraufsatz kann somit das Kultivierungsgefäß, d.h. insbesondere den Schüttelkolben, genauso steril sicher verschließen wie eine konventionelle Schüttelkolbenkappe.

Vorzugsweise stehen zwei Abgabeeinheiten 122 mit unterschiedlich großen Reservoirs 140 zur Verfügung, welche flexibel kultivierungsangepasst genutzt werden können.

Bei temperaturresistenten Feed- und pH-Korrekturlösungen können diese ebenfalls in den Reservoirs 140 vorgelegt werden und mit Deckeln 146D (siehe Fig. 8) verschlossen werden. Temperaturresistente Medien können in den Schüttelkolben vorgelegt werden. So vorbereitet kann anschließend ein Autoklavieren erfolgen, wobei hierbei die Schläuche der Reservoirs mit Adernklemmen abgeklemmt sein können, und anschließend kann ein Inokulieren in einer Sterilwerkbank erfolgen.

Es können spezielle Luer-Lock-Ports vorgesehen sein, um ein steril sicheres Arbeiten ohne Sterilarbeitsplatz zu ermöglichen. Die Luer-Lock-Ports umfassen ein Luer-Lock Ventil und ggf. einen Schlauch 160, 162 (z.B. aus PP), der durch den Deckel 146D des jeweiligen Reservoirs 140 geführt werden kann. Durch die Ports bzw. Ventile können die Reservoirs mit steril filtrierter pH-Korrektur- und Feedlösung steril befüllt werden.

Ein weiterer Port, bei dem der Schlauch 170 (z.B. aus PP) in den Schüttelkolben geführt ist, erlaubt das steril sichere Befüllen mit steril befülltem Medium, sowie das steril sichere Inokulieren. Der Schlauch 170 kann auch bis in das Medium geführt werden, z.B. für Probenahmen, Repeated-Batch-, oder kontinuierliche Kultivierungen, insbesondere auch in Verbindung mit einem weiteren Zugabe-/Entnahmeschlauch (nicht abgebildet), der an die Innenseite des Behälteraufsatzes führt.

Die Erfindung eignet sich insbesondere für Kultivierungen in einem Orbitalschüttelinkubator. Mit diesem lässt sich die Durchmischung, die Temperatur und Atmosphäre (z. B. Feuchte oder CO₂ Konz.) einstellen.

Die Halterungen 163, 173 können als Clip-Halterungen ausgebildet sein. Dies ermöglicht, insbesondere über die flexible PP-Schlauchverbindung, eine einfache Handhabung und eine Fixierung während der Kultivierung im Schüttelinkubator.

Eine Durchmischung für die Stoff- und Wärmeübertragung ist auch in Kombination von Magnetrührer und, insbesondere systemkompatiblen, Rührfisch möglich.

Bei der in Fig. 5 gezeigten Konfiguration ist die Sterilität in der Kultivierungseinheit bereits sichergestellt. Folgend kann frei von Anforderung an ein steriles Umfeld ein Elektronikmodul 132 (siehe Fig. 7) aufgesteckt werden. Anschließend kann eine Sensoreinheit 112 installiert werden. Diese kann über ein Kabel und einem Konnektor mit dem Elektronikmodul verbunden werden. Das Modul besitzt eine Verbindung 180 zur Stromversorgung und Datenübertragung.

Die Verbindung kann zur Schnittstelle des Schüttelinkubators, oder einer Zentraleinheit. In einer solchen Zentraleinheit kann Anbindung die Kultivierungseinheiten, eine Stromversorgung über Netz oder Akku und/oder ein Prozessleitsystem integriert sein. Eine Visualisierung des Prozessleitsystems kann über ein App für mobile Endgeräte erfolgen. Es besteht auch die Möglichkeit die Funktionen dieser Verbindung in das Elektronikmodul 132 zu integrieren, jedoch kann hiervon auch abgesehen werden, um das Elektronikmodul 132 kompakt und dessen Gewicht gering zu halten und eine Gefahr des Stabilitätsverlustes im Orbitalschüttler zu vermeiden

Für die Realisierung eines geschlossenen Regelkreises, mit dem in Echtzeit die Schlüsselprozessparameter steril und sicher im optimalen Bereich gehalten werden können, können in dem Elektronikmodul Aktoren 190 zur druckregulierten Dosierung integriert sein. Mit anderen Worten kann der Behälteraufsatz 100, die Abgabeeinheit 122 und/oder das Elektronikmodul 132 einen Aktor 190 umfassen, um das Nachströmen von Gas durch eine, insbesondere einen Schlauch 160, umfassende Verbindung zwischen dem Reservoir und der Innenseite des Behälteraufsatzes zu steuern. Ein solcher Aktor 190 dient insbesondere zur druckregulierten Dosierung und kann eine Spule und eine Rückholfeder umfassen, um einer Kraftübertragung auf die PP-Schläuche zu bewirken.

Die PP-Schläuche 160 führen wie beschrieben vom Deckel 146D der Reservoirs in den Schüttelkolben 50. Über den branchenüblich steril sicheren Spalt 106 zwischen Schüttelkolben 50 und Behälteraufsatz 100 kann im Schüttelkolben Atmosphärendruck herrschen. Der Auslauf 144 der Reservoirs 140 kann stark verjüngt sein.

Beim Befüllen der Reservoirs 140 über die Luer-Lock Ventile 164 werden dies bevorzugt als erste befüllt und dabei der Schüttelkolben 50 so gekippt, dass verdrängte Luft über den Reservoirs Auslauf 144 austreten kann.

Für die Regulierung der Schlüsselparameter, welche In-Situ in Echtzeit über die Sensoreinheit 112 erfasst werden können, kann nun die Dosierung der in den Reservoirs 140 vorgelegten Lösungen über die Ansteuerung der Aktoren 190 erfolgen. Bei einem zurückgestellten Aktor 190 erfolgt eine Dosierung über den Reservoirs Auslauf 144. Wird über die Aktoren 190 der PP-Schlauch zugedrückt, erfolgt keine Dosierung von Feed- oder pH-Korrekturlösung, da sich ein Unterdruck im Reservoirs ausbildet. Eine nicht dargestellt Ausführungsform sieht vor, dass ein Aktor den Reservoirauslaufs 144 steuert. Dies kann aus Platzgründen mit einem geringeren Reservoirs-Volumen verbunden sein.

## Patentansprüche

1. Behälteraufsatz (100) zum Aufsetzen auf einen Kultivierungsbehälter (50), vorzugsweise auf einen Schüttelkolben, wobei der Kultivierungsbehälter (50) einen Hals (52) und eine sich durch den Hals erstreckende Öffnung (54) aufweist, wobei der Behälteraufsatz (100) umfasst:
eine Innenseite (102), welche dem Inneren des Kultivierungsbehälters (50) zugewandt ist, wenn der Behälteraufsatz (100) auf dem Kultivierungsbehälter (50) aufgesetzt ist und eine Außenseite (104), welche dem Äußeren des Kultivierungsbehälters (50) zugewandt ist, wenn der Behälteraufsatz (100) auf dem Kultivierungsbehälter (50) aufgesetzt ist,
**dadurch gekennzeichnet, dass** der Behälteraufsatz (100)
zumindest eine Sensoreinheit (112) umfasst, wobei die Sensoreinheit (112) zumindest bereichsweise an der Innenseite (102) des Behälteraufsatzes (100) angeordnet ist, um eine Parametermessung im Inneren des Kultivierungsbehälters (50) zu ermöglichen, und
zumindest eine Abgabeeinheit (122) mit einem Reservoir (140) zur Flüssigkeitsspeicherung umfasst, wobei die Abgabeeinheit (122) zumindest bereichsweise an der Innenseite (102) des Behälteraufsatzes (100) angeordnet ist, um eine Flüssigkeitsabgabe in das Innere des Kultivierungsbehälters (50) zu ermöglichen.

2. Behälteraufsatz (100) gemäß dem vorstehenden Anspruch,
wobei der Behälteraufsatz (100) einen ringförmigen, insbesondere zylindrischen, Spalt (106) zur Aufnahme eines Halses (52) des Kultivierungsbehälters (50) aufweist und/oder
wobei der Behälteraufsatz einen Außenkranz (105) aufweist, welcher den Hals (52) des Kultivierungsbehälters (50) radial umgibt, wenn der Behälteraufsatz (100) auf dem Kultivierungsbehälter (50) aufgesetzt ist und/oder
wobei der Behälteraufsatz (100) einen Innenvorsprung (103) aufweist, welcher in den Hals (52) des Kultivierungsbehälters (50) hineinragt, wenn der Behälteraufsatz (100) auf dem Kultivierungsbehälter (50) aufgesetzt ist und
wobei der Außenkranz (105) und der Innenvorsprung (103) vorzugsweise monolithisch ausgebildet sind.

3. Behälteraufsatz (100) gemäß einem der vorstehenden Ansprüche,
wobei der Behälteraufsatz (100) senkrecht von oben auf den Kultivierungsbehälter (50) aufsetzbar ist und vorzugsweise ausgebildet ist, durch die Schwerkraft auf dem Kultivierungsbehälter (50) zu halten.

4. Behälteraufsatz (100) gemäß einem der vorstehenden Ansprüche,
wobei der Behälteraufsatz (100) eine, insbesondere am oberen Ende des Spalts (106) befindliche Anschlagsfläche (108) aufweist, welche auf dem Kultivierungsbehälter (50) zur Anlage kommt, wenn der Behälteraufsatz (100) auf dem Kultivierungsbehälter (50) aufgesetzt ist und wobei der Behälteraufsatz (100) eine von der Anschlagsfläche gemessene Höhe von weniger 10 als Zentimeter aufweist, vorzugsweise von weniger als 5 Zentimeter aufweist, noch bevorzugter von weniger als 4 Zentimeter aufweist, nochmals bevorzugter von weniger als 3 Zentimeter aufweist, besonders bevorzugt von weniger als 2 Zentimeter aufweist, ganz besonders bevorzugt von weniger als 14 Zentimeter aufweist.

5. Behälteraufsatz (100) gemäß einem der vorstehenden Ansprüche, wobei das Reservoir (140) einen oberen Reservoirabschnitt (146) und einen unteren Reservoirabschnitt (142) aufweist,
wobei der obere Reservoirabschnitt (146) eine Nachströmungsöffnung (148) zum Nachströmen von Gas aufweist und
wobei der untere Reservoirabschnitt (142) an der Innenseite (102) des Behälteraufsatzes angeordnet ist und eine Auslauföffnung (144) aufweist, um eine Flüssigkeitsabgabe durch die Auslauföffnung (144) in das Innere des Kultivierungsbehälters (50) zu ermöglichen.

6. Behälteraufsatz (100) gemäß einem der vorstehenden Ansprüche, wobei das Reservoir (140) einen oberen Reservoirabschnitt (146) und einen unteren Reservoirabschnitt (142) aufweist,
wobei der obere Reservoirabschnitt (146) breiter ist als der untere Reservoirabschnitt (142) und
wobei der obere Reservoirabschnitt (146) vorzugsweise an der Außenseite (104) des Behälteraufsatzes (100) angeordnet ist und besonders bevorzugt breiter ausgebildet ist, um einen Anschlag (147) zu bilden, wenn die Abgabeeinheit (122) und/oder das Reservoir (140) an einem Port des Behälteraufsatzes (100) installiert ist.

7. Behälteraufsatz (100) gemäß dem vorstehenden Anspruch,
wobei der Behälteraufsatz (100) und/oder die Abgabeeinheit (122) eine Verbindung zwischen der Nachströmungsöffnung (148) des oberen Reservoirabschnitts (146) und der Innenseite des Behälteraufsatzes (100) aufweist, um ein Nachströmen von Gas aus dem Inneren des Kultivierungsbehälters (50) zu ermöglichen, wenn der Behälteraufsatz (100) auf dem Kultivierungsbehälter (50) aufgesetzt ist.

8. Behälteraufsatz (100) gemäß einem der vorstehenden Ansprüche,
wobei das Reservoir (140) eine Nachfüllöffnung (149) zum Nachfüllen von Flüssigkeit aufweist und
wobei der Behälteraufsatz (100) und/oder die Abgabeeinheit (122) vorzugsweise eine Verbindung zwischen der Nachfüllöffnung (149) des Reservoirs (140) und einem an der Außenseite des Behälteraufsatzes (100) angeordneten oder anordenbaren Ventil (164) aufweist, um ein Nachfüllen von Flüssigkeit in das Reservoir (140) zu ermöglichen und
wobei der Behälteraufsatz (100) und/oder die Abgabeeinheit (122) vorzugsweise eine Halterung (163) zur lösbaren Fixierung des Ventils (164) aufweist.

9. Behälteraufsatz (100) gemäß einem der vorstehenden Ansprüche,
wobei der Behälteraufsatz (100) eine Verbindung zwischen der Innenseite (102) des Behälteraufsatzes (100) und einem an der Außenseite (104) des Behälteraufsatzes (100) angeordneten oder anordenbaren Ventil (174) aufweist, um eine Zugabe oder Entnahme aus dem Inneren des Kultivierungsbehälters (50) zu ermöglichen, wenn der Behälteraufsatz (100) auf dem Kultivierungsbehälter (50) aufgesetzt ist und
wobei der Behälteraufsatz (100) vorzugsweise eine Halterung (173) zur lösbaren Fixierung des Ventils (174) aufweist.

10. Behälteraufsatz (100) gemäß einem der vorstehenden Ansprüche,
wobei die zumindest eine Sensoreinheit (112) als Biosensoreinheit zur analytspezifischen Parametermessung ausgebildet ist und/oder
wobei die zumindest eine oder eine weitere Sensoreinheit (112) des Behälteraufsatzes (100), welche insbesondere nicht als Biosensoreinheit ausgebildet ist, als Luminophoreneinheit zur lumineszenzbasierten Parametermessung ausgebildet ist und/oder
wobei die zumindest eine oder eine weitere Sensoreinheit (112) des Behälteraufsatzes (100) als Wechselfeldeinheit zur dielektrizitätsbasierten Parametermessung ausgebildet ist und/oder
wobei die zumindest eine oder eine weitere Sensoreinheit (112) des Behälteraufsatzes (100) als Transistoreinheit zur feldeffektbasierten Parametermessung ausgebildet ist und/oder
wobei die zumindest eine Sensoreinheit (112) an einem vorderen Gehäuseabschnitt einer Sensor-Komponente (114) angeordnet oder anordenbar ist, derart, dass die zumindest eine Sensoreinheit (112) an der Innenseite des Behälteraufsatzes (100) anordenbar ist, indem die Sensor-Komponente an einem Port (110) des Behälteraufsatzes (100) installiert wird.

11. Behälteraufsatz (100) gemäß einem der vorstehenden Ansprüche,
wobei der Behälteraufsatz (100) eine Mehrzahl von Sensor-/Abgabeeinheiten (112, 122) umfasst, vorzugsweise zumindest eine Sensoreinheit (112) sowie zumindest zwei Abgabeeinheiten (122).

12. Kultivierungssystem (10) umfassend:
einen Behälteraufsatz (100) gemäß einem der Ansprüche 1 bis 11, sowie
einen, insbesondere handhaltbaren, vorzugsweise als Schüttelkolben ausgebildeten, Kultivierungsbehälter (50) zur Aufnahme eines Kulturmediums, wobei der Kultivierungsbehälter einen Hals (52) und eine sich durch den Hals erstreckende Öffnung (54) aufweist,
wobei der Behälteraufsatz (100) auf den Hals (52) des Kultivierungsbehälters (50) aufsetzbar ist, um die Öffnung (54) des Kultivierungsbehälters (50) zu verschließen, insbesondere steril zu verschließen, wobei der Behälteraufsatz (100) eine Innenseite (102) und eine Außenseite (104) aufweist, wobei die Innenseite (102) dem Inneren des Kultivierungsbehälters (50) zugewandt ist und die Außenseite (104) dem Äußeren des Kultivierungsbehälters (50) zugewandt ist, wenn der Behälteraufsatz (100) auf dem Hals (52) des Kultivierungsbehälters (50) aufgesetzt ist.

13. Kultivierungssystem (10) gemäß Anspruch 12,
wobei der Kultivierungsbehälter (50) einen flach ausgebildeten Boden (60) zum Abstellen auf einem Untergrund aufweist und/oder
wobei der Hals (52) des Kultivierungsbehälters (50) senkrecht nach oben verläuft, insbesondere, wenn der Kultivierungsbehälter (50) auf dem Untergrund abgestellt ist, und/oder
wobei der Behälteraufsatz (100) senkrecht von oben auf den Hals (52) des Kultivierungsbehälters (50) aufsetzbar ist und vorzugsweise ausgebildet ist, durch die Schwerkraft auf dem Hals (52) des Kultivierungsbehälters (50) zu halten.

14. Kultivierungssystem (10) gemäß einem der Ansprüche 12 oder 13,
wobei der Kultivierungsbehälter (50) ein Volumen von weniger als 2801 Milliliter aufweist, vorzugsweise von weniger als 1801 Milliliter aufweist, noch bevorzugter von weniger als 501 Milliliter aufweist, nochmals bevorzugter von weniger als 251 Milliliter aufweist, besonders bevorzugt von weniger als 126 Milliliter aufweist und/oder
wobei der Kultivierungsbehälter (50) einen Durchmesser von weniger als 22 Zentimeter oder von weniger als 21 Zentimeter aufweist, vorzugsweise von weniger als 14 Zentimeter oder von weniger als 12 Zentimeter aufweist, besonders bevorzugt von weniger als 9 Zentimeter oder von weniger als 8 Zentimeter aufweist und/oder
wobei der Kultivierungsbehälter (50) eine Höhe von weniger als 31 Zentimeter aufweist, vorzugsweise von weniger als 23 Zentimeter aufweist, nochmals bevorzugter von weniger als 19 Zentimeter aufweist, besonders bevorzugt von weniger als 17 Zentimeter aufweist, ganz besonders bevorzugt von weniger als 14 Zentimeter aufweist.

15. Verfahren zur Vermehrung oder Kultivierung biologischen Materials vorzugsweise zur Herstellung von Pharmazeutika, insbesondere Biopharmazeutika, umfassend:
Bereitstellen eines Kultivierungssystems (10) gemäß einem der Ansprüche 11 bis 14,
Bereitstellen einer Feedlösung, insbesondere enthaltend die für die Produktbildung erforderlichen Substrate, z.B. Saccharide, wobei die Feedlösung in dem Reservoir der Abgabeeinheit (122) bereitgestellt wird,
Bereitstellen einer pH-Korrekturlösung, insbesondere zum Anheben des pH-Werts, z.B. enthaltend NaOH, wobei die pH-Korrekturlösung vorzugsweise in einem weiteren Reservoir bereitgestellt wird und/oder in der Feedlösung enthaltend bereitgestellt wird, und
wobei mit der Sensoreinheit (112) in Echtzeit ein Parameter zur Prozesskontrolle erfasst wird und mit der Abgabeeinheit (122) eine aktive Korrektur, z.B. pH-Korrektur oder Feedrate, erfolgt.
